# EUROPÄISCHE PATENTSCHRIFT

(11) **EP 0 547 429 B1**
(45) Veröffentlichungstag und Bekanntmachung des Hinweises auf die Patenterteilung: **19.06.1996**
(21) Anmeldenummer: 92120525.8
(22) Anmeldetag: 02.12.1992
(51) Int. Cl.: A61M 15/00

(54) **Pulverinhalator**
Powder inhaler
Inhalateur de poudres

(30) Priorität: 14.12.1991 DE 4141363; 06.04.1992 DE 4211475
(43) Veröffentlichungstag der Anmeldung: 23.06.1993
(73) Patentinhaber: ASTA Medica Aktiengesellschaft, D-01277 Dresden (DE)
(72) Erfinder: Lerk, C.F., Prof.Dr. Ir., NL-9321 GG Peize (NL); de Boer, Anne H., NL-9204 AG Drachten (NL)

(56) Entgegenhaltungen:
- EP-A- 0 211 595
- EP-A- 0 406 893
- EP-A- 0 407 028
- EP-A- 0 424 790
- FR-A- 2 447 725
- US-A- 2 581 182

## Beschreibung

Die Erfindung betrifft einen Pulverinhalator gemäß dem Oberbegriff des Anspruchs 1, wie es beispielsweise in der FR-A-2 447 725 beschrieben ist.

In der inhalativen Asthmatherapie werden heute immer häufiger Pulverinhalatoren als Dosier- und Inhaliergeräte eingesetzt, da die bisher üblichen Dosieraerosole wegen der Verwendung von halogierten Kohlenwasserstoffen als Treibgas aus Umweltschutzgründen nicht mehr eingesetzt werden können.

Dosieraerosole haben weiter den Nachteil, daß der Patient in der Lage sein muß, Sprühstoßauslösung und Inhalation zu koordinieren. Dies ist unter den Bedingungen eines akuten Asthmaanfalls, bei dem der Patient unter starkem Streß steht, nicht immer der Fall. Bei Pulverinhalatoren entfällt die Notwendigkeit der Koordination von Einatmung und Sprühstoßauslösung, da die Einatemluft des Patienten den Wirkstoff mitführt.

In den herkömmlichen Pulverinhalatoren (zum Beispiel nach EP-A 406 893) ist jede Dosis des Wirkstoffs in einer einzelnen Hartgelatinekapsel enthalten. Die Kapsel wird in das Gerät eingelegt, durch mehrere Dornen zerstochen und der freigesetzte Inhalt wird durch den Einatemluftstrom des Patienten mitgetragen und gelangt in die Lunge des Patienten.

Nachteilig bei den Pulverinhalatoren mit Kapselfüllung ist, daß zur besseren Füllung der Kapsel und zum Erhalt der dosiergenauigkeit ein inerter Hilfsstoff, wie zum Beispiel Lactose, hinzugefügt werden muß. Bei einigen Patienten kann das Einatmen des feinverteilten Lactosepulvers zu Irritationen der Luftwege führen.

Weiterhin ist nicht gewährleistet, daß die zerstochene Kapsel vollständig geleert wird und ihr Inhalt dem Patienten zur Verfügung steht. Ebenso besteht die Gefahr, daß Kapselbruchstücke mit eingeatmet werden können.

DE-A-27 04 574 beschreibt einen Inhalator für in Kapseln eingeschlossenes Pharmakon, wobei die Kapseln in einem drehbaren Magazin gelagert sind, das durch Drehen der äußeren Hülle des Inhalators weiterbewegt wird.

Geräte, die ohne Kapseln auskommen, vermeiden die oben beschriebenen Nachteile. So wird zum Beispiel beim Turbuhaler® der Firma Astra ohne den Hilfsstoff Lactose gearbeitet und der Arzneimittelwirkstoff direkt inhaliert.

Jedes Gerät erlaubt die Entnahme von zweihundert Dosierungen. In einem Vorratsbehälter befindet sich das Pharmakon in Form sphärischer Aggregate eines mikronisierten Pulvers. Beim Inhalieren zerfallen diese Aggregate in das mikronisierte Pulver, welches lungengängig ist.

Die Dosierung des Pulvers erfolgt mit Hilfe einer drehbaren Dosierscheibe. Das sphärische Aggregat des Pharmakons wird von oben in eine Gruppe von vier Meßöffnungen gepreßt, die die Menge des zu inhalierenden Granulats festlegen. Die Abmessung des Granulats erfolgt volumetrisch.

Die Dosierscheibe wird weitergedreht und gelangt mit den vier gefüllten Dosierlöchern in einen Teil des Einatemluftstroms und der Wirkstoff wird aus den Dosierlöchern gerissen. In der spiralförmigen Strömung des Mundstücks werden die sphärischen Aggregate zerliegt und als mikronisiertes Pulver eingeatmet.

Nachteilig beim Turbuhaler® ist, daß im Laufe der Zeit die Dosierlöcher verstopfen können und die Dosiergenauigkeit abnimmt und zwar umsomehr, Je länger das Gerät im Gebrauch ist. Weiter sammelt sich im Laufe der Zeit eine nicht unerhebliche Pharmakonmenge im Mundstück an. Dies stellt ein Risiko für den Patienten dar, da die Gefahr besteht, daß er eine zu große Pharmakonmenge auf einmal einatmet.

EP-A-407 028
beschreibt einen Pulverinhalator, der von einem kompakten Arzneimittelvorrat mittels eines Schabers eine abgemessene Menge abschabt und vom Luftstrom des Patienten diese Menge in eine Zyklonkammer überführen läßt. Das im Zyklon gesichtete Gemisch wird vom Patienten eingeatmet.
EP-A-387 222
beschreibt eine elektronisch gesteuerte Medikamentfreisetzungseinrichtung, die auf das Strömgeräusch der eingeatmeten Luft anspricht und eine Dosis aus dem Medikamentvorrat freisetzt.
EP-A-237 507
beschreibt einen Pulverinhalator mit im Luftstrom angeordneten Prallflächen, die den aus dem Dosierbehälter freigesetzten Arzneimittelvorrat zerlegen.
EP-A-69 715
beschreibt einen durch den Einatemluftstrom des Patienten aktivierten Pulverinhalator, wobei der Einatemluftstrom aus einer Dosiereinrichtung, die in den Einatemluftstrom gedreht werden kann, das volumetrisch abgemessene Pharmakon mitreißt.

FR-A-2 447 725
beschreibt einen Pulverinhalator aus zwei übereinander angeordneten Gehäuseteilen, wobei das die Wirkstoffdosierungen enthaltende Gehäuseunterteil um seine Längsachse drehbar gegen das Gehäuseoberteil gelagert ist.

Aufgabe der Erfindung ist es, dem Patienten eine über lange Zeit dosiergenaue Inhalierhilfe zur Verfügung zu stellen, die einfach zu bedienen und leicht zu reinigen ist, Koordinationsprobleme zwischen der Medikamentenfreisetzung und der Einatmung nicht auftreten läßt und ohne die Zufuhr von Fremdenergie auskommt.

Die Lösung dieser Aufgabe erfolgt bei einem Pulverinhalator der eingangs genannten Art erfindungsgemäß mit den im Kennzeichen des Anspruchs 1 angegebenen Merkmalen.
Vorteilhafte Ausbildungsformen dieses Pulverinhalators sind in den Unteransprüchen beansprucht.

Bei dem erfindungsgemäßen Pulverinhalator steht die Dosis sofort zur Verfügung, ohne daß erst umständlich eine Hartgelatinekapsel in das Gerät eingelegt werden oder eine Blisterfolie durchstochen werden muß.

Nach Entleerung der vorgefüllten Dosierkammern wird eine neue Patrone mit gefüllten Dosierkammern eingesetzt. Die mechanische Vorrichtung des Pulverinhalators kann mehrmals verwendet werden.

Die Wirkstoffpartikel, die der Asthmapatient einatmet, enthalten praktisch keine größeren Bestandteile oder Agglomerate. Die Partikel haben zu 90 - 95 % eine lungengängige Größe. Dadurch wird weniger Wirkstubstanz im Mundstück, im Mund oder im Rachen niedergeschlagen. Die Gefahr, daß aus dem Mundstück eine hohe, den Patienten gefährdende Dosis freigesetzt wird, ist beseitigt.

Durch die Umhüllung des partikeltragenden Luftstroms mit einem partikelfreien Luftstrom wird zusätzlich die Niederschlagung des Pharmakons im Mund-Rachenraum erheblich vermindert.
Die Häufigkeit lokaler Nebenwirkungen im Mund- und Rachenraum wird reduziert und die Wirksamkeit der Medikation wird erhöht.

Zur Freisetzung der Dosis und zum Zerlegen der Wirkstoffmischung in lungengängige Partikel dient als Energiequelle die kinetische Energie des Einatemluftstroms des Patienten, der vom inhalierenden Patienten erzeugt wird. Eine externe Energiequelle, zum Beispiel eine Batterie wie in WO 90/13328 oder WO 90/13327 offenbart, wird nicht benötigt.

Die hohe Dosiergenauigkeit über alle Dosierungen wird dadurch erreicht, daß für jede Dosierung des Pharmakons eine eigene, vom Hersteller mit Wirkstoffmischung gefüllte Dosierkammer zur Verfügung steht.
Fehlfüllungen durch Zusammenklumpen des Granulats im Vorratsbehälter oder durch Reste der vorherigen Dosierung in der Dosierscheibe, wie sie bei Geräten mit Dosierscheibe auftreten können, sind mit diesem Verfahren ausgeschlossen.

Durch wechselnde Lufttemperaturen und -feuchtigkeiten bedingtes Zusammenklumpen des Arzneimittelvorrats wird durch die erfindungsgemäße Konstruktion ebenfalls verhindert, da die Dosierscheiben durch Positionierung im Zwischenraum zwischen den Zylindern zuverlässig von der Außenluft abgeschlossen sind.

### Figurenbeschreibung:

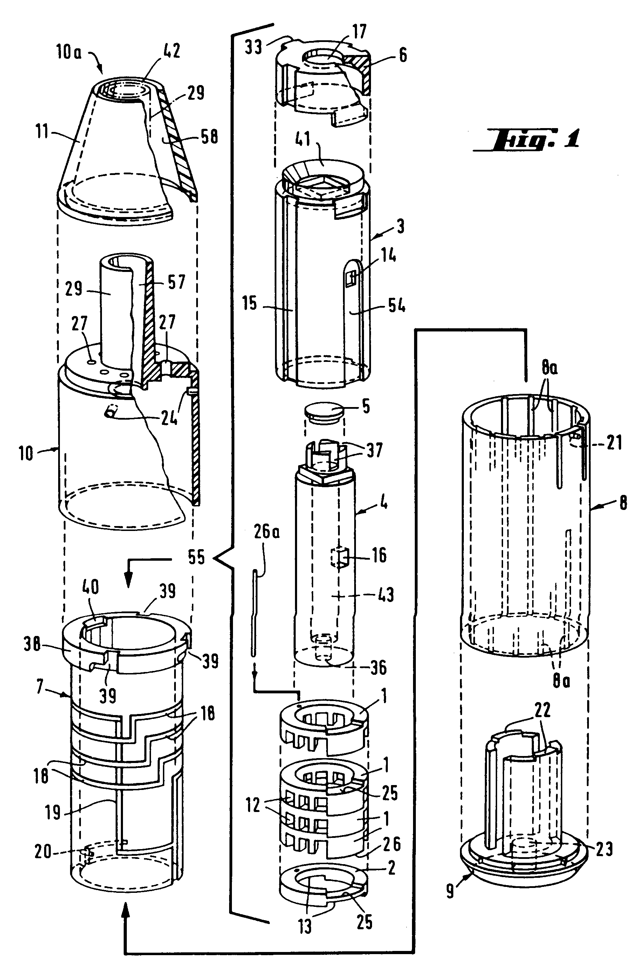

Figur 1 stellt eine Explosions-Zeichnung des gesamten Inhalators gemaß Beispiel 1 dar.

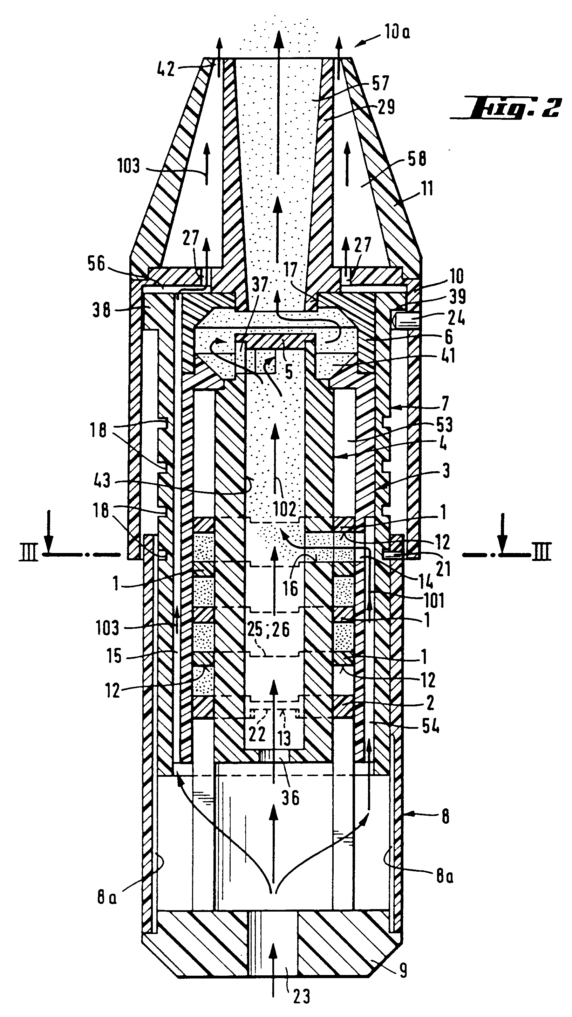

Figur 2 zeigt den Längsschnitt des Inhalators gemäß Beispiel 1 mit den Luftströmen.

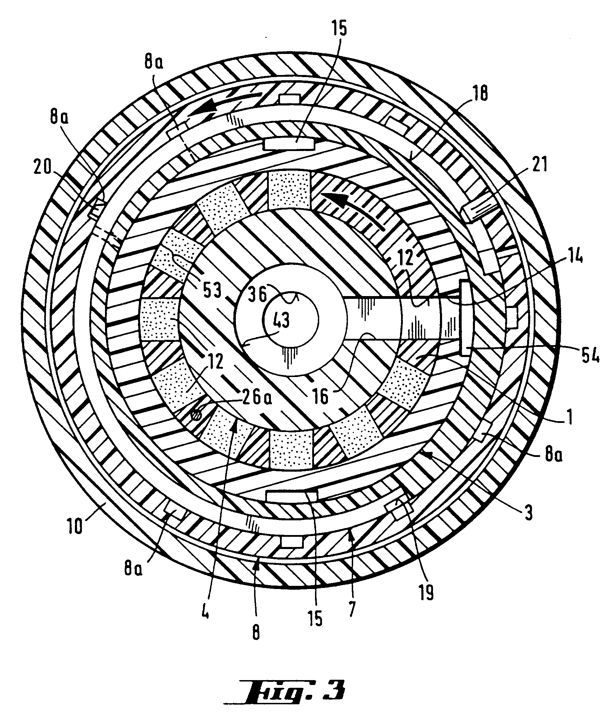

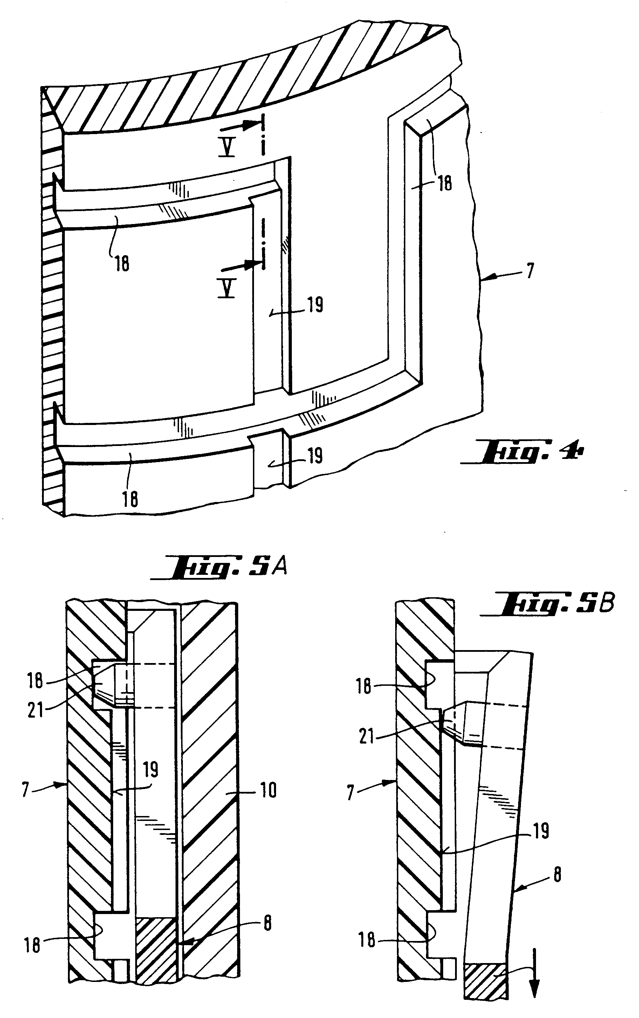

Figur 3 zeigt den Querschnitt entlang der Achse III - III von Figur 2.

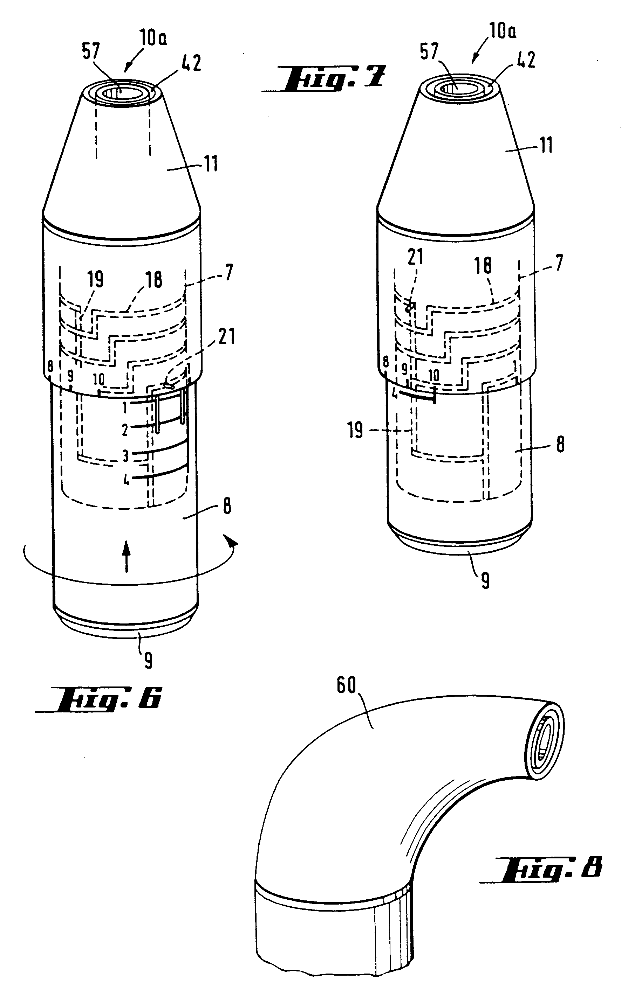

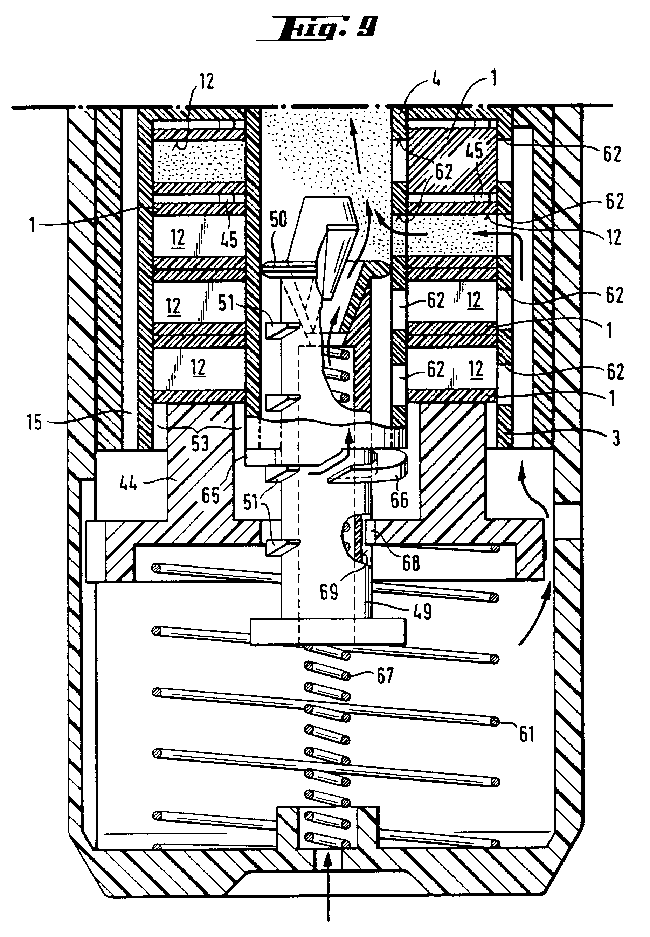

Figur 4 zeigt die Führungsrillen.

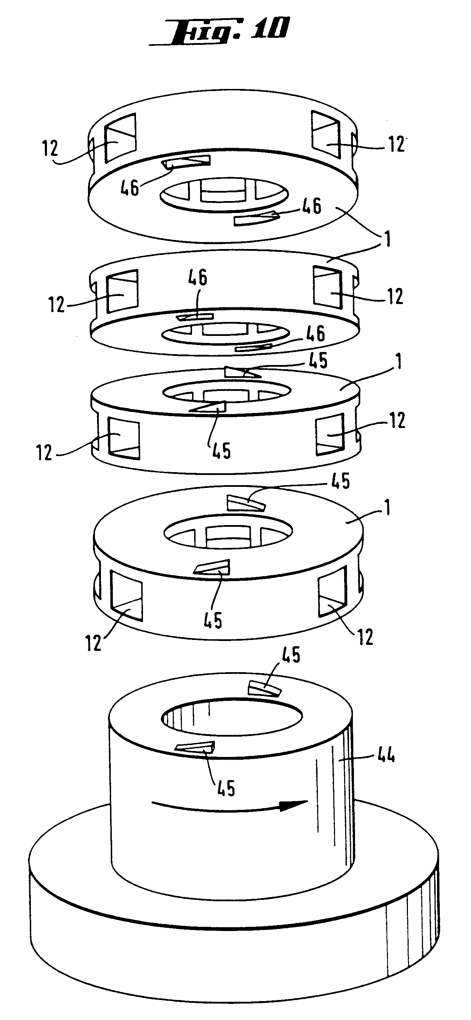

Figur 5 A und 5 B veranschaulicht den Mechanismus in den Führungsrillen.

Figur 6 zeigt den Inhalator im gefüllten Zustand.

Figur 7 zeigt den Inhalator im leeren Zustand.

Figur 8 zeigt das Mundstück.

Figur 9 zeigt den Längsschnitt durch den Inhalator mit dem Absperrkolben.

Figur 10 zeigt den Mechanismus der sich gegenseitig antreibenden Dosierscheiben.

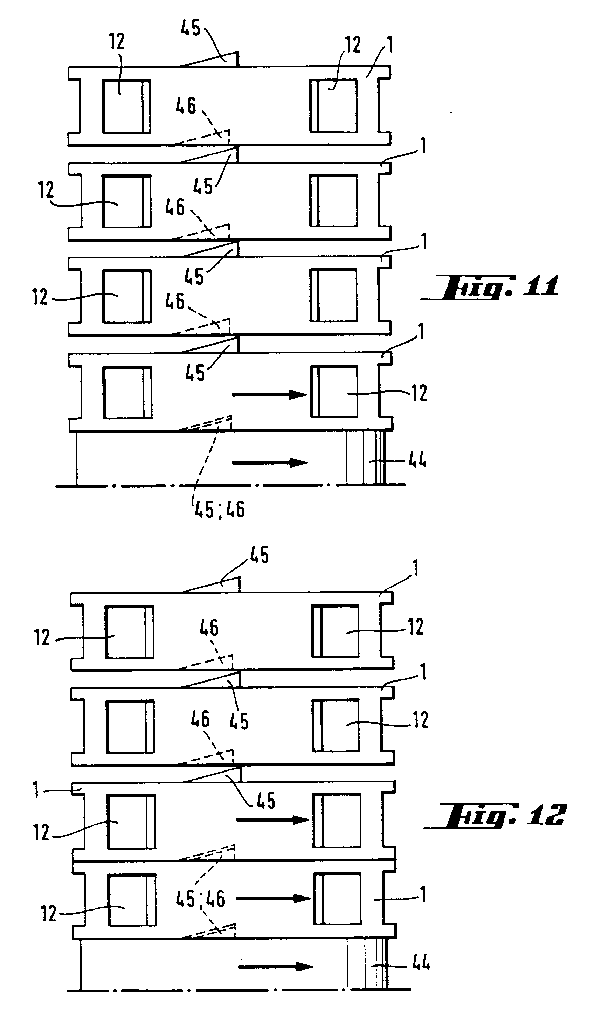

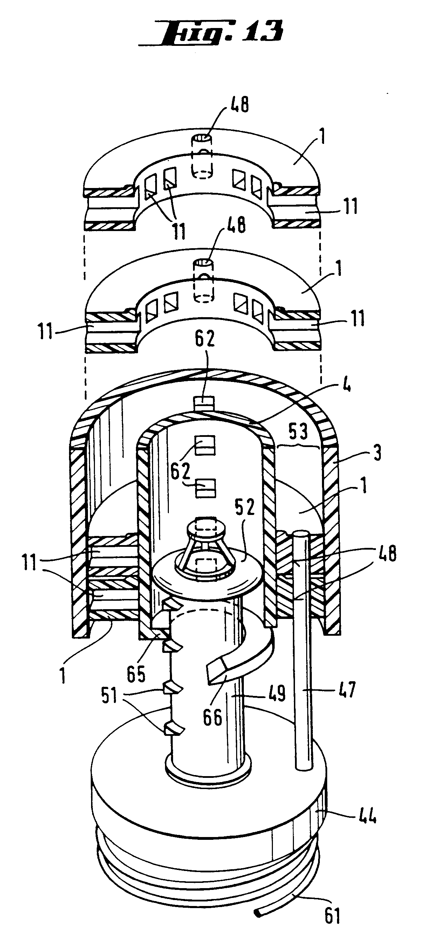

Figur 11 und 12 zeigen Details der sich antreibenden Dosierscheiben.

Figur 13 stellt eine Explosionszeichnung von Beispiel 3 dar.

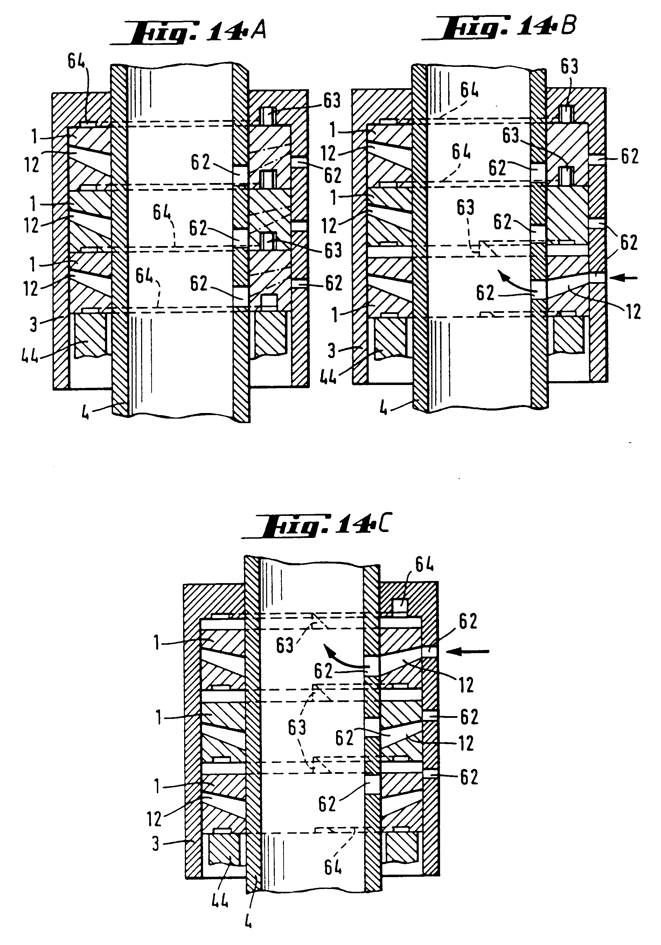

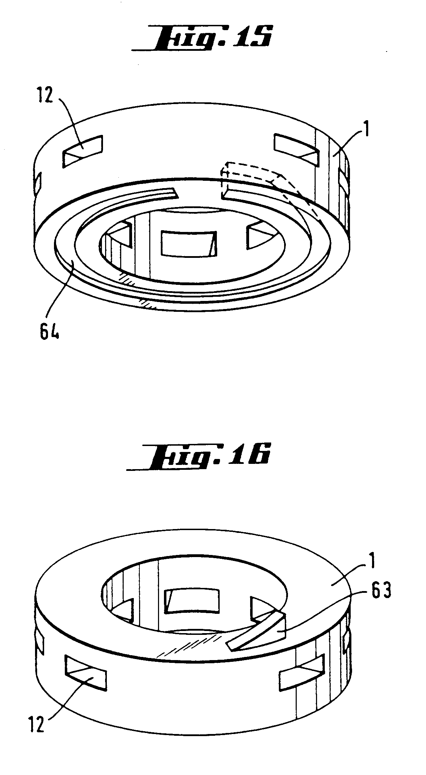

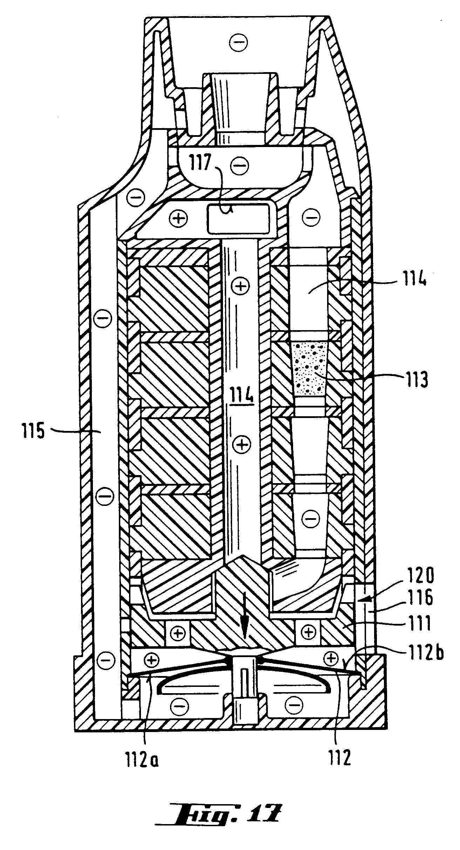

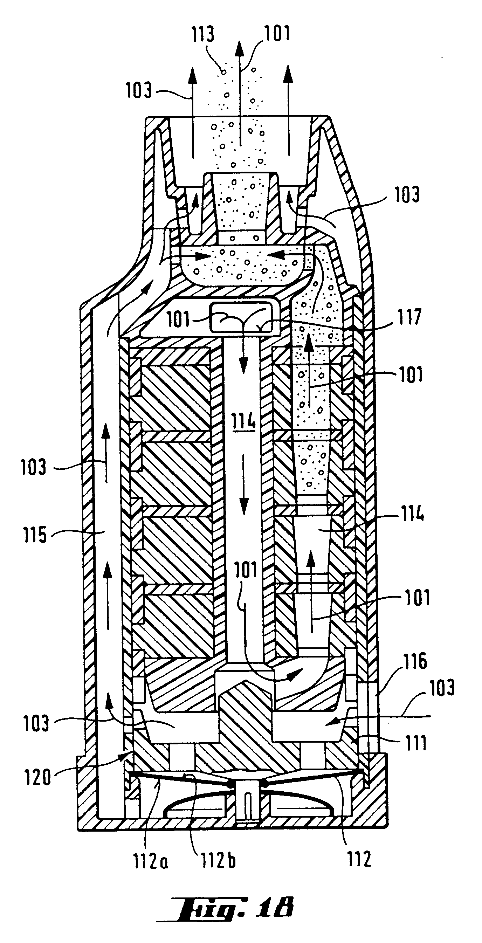

Figuren 14 A-C stellen die Konstruktion gemäß Beispiel 4 dar.

Figuren 15 und 16 zeigen die Dosierscheiben von Beispiel 4.

Figur 17 und 18 verdeutlichen die Regelung des Luftstromes gemäß Beispiel 5.

Da die Asthmapatienten den Inhalator ständig bei sich tragen, um beim Auftreten von Atemnot sofort inhalieren zu können, ist neben dem Schutz vor Luftfeuchtigkeit ebenso ein Schutz vor mechanischen Erschütterungen notwendig, um zu verhindern, daß der Wirkstoff aus den Dosierkammern (12) geschleudert wird. Dies wird ebenfalls durch den Einschluß der Dosierscheiben (1) im Zwischenraum (53) erreicht.

Die Erfindung betrifft einen Pulverinhalator zur mehrfachen Applikation pulverförmiger Arzneistoffe in vorgefüllten Dosierkammern.

Der Pulverinhalator besteht in der erfindungsgemäßen Ausführung aus vier Hauptteilen:
dem Einwegdosierzylinder (55) aufgebaut aus den Teilen 1, 2, 3, 4, 5 und 6, dem Führungszylinder (7), dem Außenzylinder (8) mit dem Inhalatorboden (9) und dem Mundstück (10, 11, 29). In diese Teile kann der Patient den Pulverinhalator zur Reinigung und zum Nachfüllen auseinandernehmen.

Der Einwegdosierzylinder (55) besteht aus einem inneren Dosierzylinder (4) mit Deckel (5) mit einer Lufteintrittsöffnung (36) am Boden für die Zuführung eines Teils der Inhalationsluft und mit einer Durchtrittsöffnung (16) an der Seitenwand für die Durchführung eines weiteren Teils des Luftstroms. Am oberen Ende seines inneren Kanals (43) ist ein Deckel (5) angebracht, der bewirkt, daß die angesaugte Inhalationsluft nur in tangentialer Richtung durch tangentiale Luftführungsschlitze (37) in eine Zyklonkammer (41) abfließen kann.

Der innere Dosierzylinder (4) wird von einem äußeren Dosierzylinder (3) derart umschlossen, daß eine Verdrehung der beiden Zylinder gegeneinander unmöglich ist.

Dies wird durch formschlüssige Verbindung des inneren Dosierzylinders (4) mit dem äußeren Dosierzylinder (3) an der Oberseite der Zylinder erreicht.
Der äußere Dosierzylinder (3) hat fluchtend mit der Durchtrittsöffnung (16) des inneren Dosierzylinders (4) ein seitliches Lufteinlaßloch (14) für die Inhalationsluft.

Der die Feinstpartikel enthaltende Inhalationsluftstrom wird aus dem inneren Kanal (43) des inneren Dosierzylinders (4) durch die tangential angeordneten Luftführungsschlitze (37) sofort in die Zyklonkammer (41) geleitet.

Dort werden durch die Zentrifugalkräfte die eventuell noch bestehenden Agglomerate durch Stöße gegeneinander und durch Stöße an der Wand der Zyklonkammer und am Deckel (5) in lungengängige Partikel zerlegt und über die Öffnung (17) zum Ausströmrohr (29) des Mundstücks (10) geleitet.
Im Zwischenraum (53) zwischen äußerem Dosierzylinder (3) und innerem Dosierzylinder (4) sind die vier Dosierscheiben (1) und die Endscheibe (2) angeordnet, die das Wirkstoffmagazin bilden.

### Beispiel 1

Die Dosierscheiben (1) und die Endscheibe (2) können sowohl aus einzelnen Scheiben aufgebaut als auch aus einem Teil gefertigt sein. Die Scheiben greifen so ineinander,
daß alle vier Dosierscheiben (1) gleichzeitig und gleichmäßig gedreht werden können.

Die Verdrehverriegelung der einzelnen Dosierscheiben (1) nebst der Endscheibe (2) kann durch sich ergänzende Nuten (25) und untere Dosierscheibenvorsprünge (26) erfolgen.
Ein zusätzlicher Stahlstift (26 a) kann die Stabilität weiter erhöhen. Die einzelnen Dosierscheiben (1) nebst Endscheibe (2) können auch mit Hilfe der Spritzgußtechnik aus einem einheitlichen Stück gefertigt werden. Die vier Dosierscheiben (1) sind mit Aussparungen versehen, so daß beim Stapeln Dosierkammern (12) entstehen, die eine bestimmte Menge Pharmakonpulver oder eine Mischung aus Wirkstoffen und Hilfsstoffen aufnehmen können.

Der Einwegdosierzylinder (55), aufgebaut aus den Dosierscheiben (1), der Endscheibe (2), dem inneren Dosierzylinder (4), dem äußeren Dosierzylinder (3) und dem Zyklonkammergehäuse (6), ist im Führungszylinder (7) gelagert.
An der Oberseite besitzt der Führungszylinder einen Flansch (38) mit zum Beispiel eingearbeiteten Nutensystemen (39), welche mit im Mundstück (10) angeordneten Mundstückvorsprüngen (24) einen Bajonettverschluß bilden, mittels welchem der Führungszylinder (7) mit dem Mundstück (10) verbunden werden kann. Anstelle dessen kann auch eine Schnappverbindung vorgesehen sein.

Am oberen Rand des Führungszylinders (7) ist eine Flanschaussparung (40) vorgesehen, damit der Einwegdosierzylinder (55) mit Hilfe eines an dem Zyklonkammergehäuse (6) angebrachten Zyklonvorsprunges (33) in eindeutiger Lage einrastet und fehlerhaftes Zusammensetzen durch den Patienten vermieden wird.

Der Führungszylinder (7) enthält an der Außenseite Führungsnuten (18) und Steuerkurven (19) für die Führung des Außenzylinders (8) und des Inhalatorbodens (9) mit Hilfe einer Führungsnocke (21). Am unteren Rand des Führungszylinders (7) befindet sich eine Ratschennase (20), die nach jeder Verdrehung des Inhalatorbodens um 30 Grad in einer Furchnut(8 a) des Außenzylinders (8) einrastet.

Der Inhalatorboden (9) besitzt an seiner Oberseite zwei halbzylinderförmige Antriebsstücke (22), die in Vertiefungen (13) der Endscheibe (2) des Dosierscheibenstapels eingreifen.

Im Inhalatorboden (9) befindet sich eine Eintrittsöffnung (23) zur Luftdurchführung für die mit Wirkstoff zu beladende Luft, für den Falschluftstrom und für den Mantelstrom.
Das Mundstück (10) wird mit einem Bajonettverschluß am Flansch (38) des Führungszylinders (7) befestigt. Das Mundstück (10) besteht aus zwei leicht auseinandernehmbaren Teilen. Es enthält ein innenkonisches Aussfrömrohr (29), umgeben von einer Mundstückhülle (11). Zwischen Zyklonkammergehäuse (6) und Mundstückhülle (11) befindet sich ein Zwischenraum (56), durch den Luft strömen kann. Die konische Ausbildung des Mundstückkanals (57) im Mundstück (10) verhindert eine zweite Zyklonwirkung. Dadurch wird erreicht, daß sich wesentlich weniger Pharmakon an den Wänden des Mundstücks niederschlägt als mit Zyklonwirkung im Mundstück ohne Ausströmrohr (29). Das Mundstück kann auch als gebogenes Mundstück (60) ausgebildet sein.

Der Inhalatorboden (9) wird in axialer Richtung geschoben, bis die Führungsnocke (21) die erste Führungsnut (18) im Führungszylinder (7) erreicht.

Gleichzeitig wird zwischen den Antriebsstücken (22) des Inhalatorbodens (9) und der Endscheibe (2) des Dosierzylinderstapels formschlüssiger Kontakt hergestellt, so daß durch Verdrehen des Inhalatorbodens (9) die Endscheibe (2) und die Dosierscheiben (1) gedreht werden können.

Eine mit Pulver gefüllte Dosierkammer (12) wird vor das Lufteinlaßloch (14) im äußeren Dosierzylinder (3) gedreht und kann nun über die im inneren Dosierzylinder (4) fluchtend eingebrachte Durchtrittsöffnung (16) mittels Partikelaustragsstrom (101) in den inneren Kanal (43) hinein entleert werden. Der durch die Lufteintrittsöffnung (36) geführte Falschluftstrom (102) befördert dann das Pulver entsprechend weiter.

Ab diesem Punkt der Drehung ermöglicht die Ratschennase (20) nur das Weiterdrehen nach rechts.
Nach dem Inhalieren der ersten Dosis aus einer Dosierkammer (12) kann der Patient die zweite und die folgenden Dosen vor die Durchtrittsöffnung (16) des inneren Dosierzylinders (4) drehen.
Nach dem Inhalieren der letzten Dosis der ersten Dosierscheibe (1) im Wirkstoffmagazin dreht der Patient weiter, bis die Blockade der Führungsnocke (21) durch das Ende der ersten Führungsnut (18) ein Weiterdrehen und damit ein erneutes Positionieren einer schon geleerten Dosierkammer (12) vor die Durchtrittsöffnung (16) verhindert.

Die Führungsnocke (21) greift so in die Führungsnuten (18) auf dem Führungszylinder (7) ein, daß der Außenzylinder (8) und der durch Klemmsitz befestigte Inhalatorboden (9) mit den Antriebsstücken (22) im Eingriff in die Vertiefung (13) der Endscheibe (2) der Dosierscheiben (1) die mit Wirkstoff oder mit einer Mischung aus Wirkstoff und Hilfsstoff gefüllten Dosierkammern (12) der entsprechenden Dosierscheiben (1) vor die Durchtrittsöffnung (16) führt.

Der Inhalatorboden (9) mit Außenzylinder (8) kann nur noch in axialer Richtung verschoben (axialer Bereich der Führungsnut (18)) werden, bis die Führungsnocke (21) die Führungsnut (18) für die Umdrehung der zweiten Dosierscheibe (1) erreicht. Durch Rechtsdrehung können die weiteren Dosierungen aus den Dosierkammern (12) entnommen werden.

In der durch die Zeichnungen näher erläuterten Ausführungsform sind zum Beispiel vier Dosierscheiben (1) übereinander angebracht. Anschließend werden die dritte und die vierte Dosierscheibe entleert.

Nach der Entleerung der letzten Dosierkammer (12), im Beispiel ist es die vierzigste, kann das Inhalatorunterteil (8, 9) weder weitergedreht noch in axialer Richtung weitertransportiert werden. Die Weiterdrehung nach rechts wird durch die obere Führungsnocke (21) und durch die Führungsnut (18) (Ende der Nut) verhindert.

Die Rückdrehung des Inhalatorunterteils (8, 9) wird durch die Ratschennase (20) im unteren Bereich des Führungszylinders (7) verhindert, welche in eine entsprechende Furchnut (8 a) des Außenzylinders (8) eingerastet ist. Das axiale Abziehen des Inhalatorunterteils (8, 9) entlang der Steuerkurve (19) wird durch die geringere Nuttiefe derselben gegenüber der Führungsnut (18) verhindert, derart, daß das zum Übertritt der Führungsnocke(21) von der tieferen Führungsnut(18) in die weniger tiefe Steuerkurve (19) erforderliche Auslenken der Führungsnocke (21) durch die innere Mantelfläche des Mundstücks (10) verhindert wird. Erst nach Abnehmen des Mundstücks (10) (Bajonett 24/39) kann die Führungsnocke (21) unter Auslenkung derselben in die Steuerkurve (19) einfahren, beziehungsweise das Inhalatorunterteil (8, 9) axial abgenommen werden.
Ein neuer Einwegdosierzylinder (55) kann nach der Reinigung und Trocknung des Mundstücks (10) wieder angebracht werden.

Anschließend wird der Inhalator wieder zusammengebaut und in die Position gedreht, die die Inhalation der ersten Dosierung erlaubt.

In einer weiteren, bevorzugten Ausfühungsform des Dosierscheibenantriebes werden die Dosierscheiben nicht durch einen Mechanismus gemeinsam bewegt, sondern treiben sich nacheinander durch Mitnehmereinrichtungen an.

Der Einwegdosierzylinder besteht wie vorstehend beschrieben aus einem inneren und äußeren Dosierzylinder, der ringförmige Zwischenraum zwischen den Zylindern nimmt die Dosierscheiben auf.

Jede Dosierscheibe kann eine "tote" oder nicht-aktivierte Stellung einnehmen. Die Breite der nicht-aktivierten Stellung ist so bemessen, daß die Abdichtung der nicht mehr benötigten Ausströmöffnungen (14, 16) zuverlässig gewährleistet ist.

Bei beispielsweise sieben Dosierungen pro Dosierscheibe beträgt der zu Abdichtungszwecken zur Verfügung stehende Teil des Drehwinkels 45 °. Am unteren Ende des Dosierungsstapels ist die Antriebsscheibe (44) für die Drehbewegung der Dosierscheiben angeordnet.
Die Ausblasöffnung im inneren Zylinder führt direkt in die Zerlegeeinrichtung (Zyklonkammer (41) oder Prallplatte).

### Beispiel 2

In einer beispielhaften Ausführungsform der Erfindung trägt die Dosierscheibe an ihrer Oberseite zwei keilförmige Nocken (45), die entlang von Kreisbögen mit verschiedenen Radien angeordnet sind.

Wenn die Dosierscheiben (1) gedreht werden, besteht nur an der Spitze der keilförmigen Nocke (45) ein Kontakt mit der Unterseite der nächsten Dosierscheibe (1), so daß nur eine minimale Reibungskraft übertragen wird, diese Reibungskraft ist gegenüber den Reibungskräften, die von der Wand des äußeren Zylinders auf die Dosierscheibe (1) ausgeübt werden, vernachlässigbar klein.

Daher wird die folgende Dosierscheibe (1) erst dann bewegt, wenn die keilförmigen Nocken (45) der ersten Dosierscheibe (1) in die passenden keilförmigen Schlitze (46) der zweiten Dosierscheibe (1) eingreifen. Durch das Ineinandergreifen der Dosierscheiben (1) verringert sich ihr Abstand, diese Längenverminderung und das Ineinanderrasten werden durch eine Spiralfeder (61) unterstützt, die die Dosierscheiben (1) zusammendrückt.
Jede Dosierscheibe kann beispielsweise um 315 ° gedreht werden, um sieben vorgefüllte Kammern (12) zu entleeren.

Die Luftdurchführungsöffnungen (62) sind vorteilhaft als einzelne Öffnungen und nicht als Schlitz ausgebildet, um die Zylinder nicht unnötig strukturell zu schwächen. Ein steifes Gehäuse ist notwendig, um das Arzneimittelpulver in den Dosierscheiben (1) zuverlässig vor Feuchtigkeit zu schützen.

Da die Dosierscheiben (1) nacheinander geleert werden müssen, ist es notwendig, nicht in Gebrauch befindliche Scheiben vom Luftstrom abzusperren. Dies wird durch die tote Stellung der sich nicht drehenden Dosierscheiben (1) erreicht. Die tote Stellung der vorgefüllten Dosierscheiben (1) wird dadurch bewerkstelligt, daß über einen gewissen Umfang der Dosierscheiben (1) keine Dosierkammern (12) angeordnet werden, so daß der Werkstoff der Dosierkammer dicht an der Zylinderinnenwand anliegt und so den Luftstrom absperrt.

Für die geleerten und sich mitdrehenden Dosierscheiben (1) gilt dies jedoch nicht mehr, da die bereits geleerten Dosierkammern (12) synchron vor die Luftdurchführungsöffnungen (62) geführt werden.

Dies ist nachteilig, da der Luftstrom, der durch die vorgefüllte Kammer bläst, nicht mehr ausreicht, um das Arzneimittel aus der Kammer auszutragen. Die Reproduzierbarkeit der Dosis ist nicht mehr gewährleistet.

Um die nicht erwünschte Luftströmung durch die bereits geleerten Dosierkammern zu unterdrücken, werden beispielsweise folgende Maßnahmen ergriffen:
Ein axial verschiebbarer, durch die Feder (67) beaufschlagter zylindrischer Kolben (49) mit einem am oberen Ende angebrachten Dichtelement (50) wird synchron zu den Dosierscheiben (1) nach oben bewegt und sperrt die unter dem Dichtelement (50) liegenden Dosierscheiben (1) vom Luftstrom ab.
Die Eintauchtiefe des Kolbens in den inneren Hohlraum des Zylinders wird durch eine Anzahl von keilförmigen Kolbenvorsprüngen (51) an der äußeren Kolbenwand und durch Führungsvorrichtungen (65, 66) an dem Boden des inneren Zylinders bestimmt.

Die Führungsvorrichtung (65, 66) besteht aus einem Flansch mit dem gleichen Außendurchmesser wie der Hohlraum des Innenzylinders und einem kleineren Innendurchmesser, er verhindert das Vorbeigleiten der keilförmigen Kolbenvorsprünge (51) an der äußeren Kolbenwand.
Die Führungsvorrichtung (65) ist nur über den halben Umfang am Innenzylinder befestigt, der restliche Umfang wird durch den freihängenden Kolbenflansch (66) abgedeckt.

Der freibewegliche Kolbenflansch (66) der Führungsvorrichtung (65) besitzt ein abgeschrägtes Ende. Wenn nun die Antriebsscheibe (44) und der Kolben (49) (mittels Antriebsscheibenvorsprung (68) und Feder (67) drehfest aber axialverschiebbar miteinander verbunden) gedreht werden, dreht sich er erste keilförmige Kolbenvorsprung (51) entlang des festen Teils der Führungsvorrichtung (65) und erreicht nach Entleeren der Kammern in der ersten Dosierscheibe den Schlitz, der durch den freihängenden Teil des Kolbenflansches (66) gebildet wird.

Der keilförmige Kolbenvorsprung (51) gleitet durch den Schlitz und erlaubt die Verschiebung des Kolbens (49) durch Federkraft um die Höhe einer Dosierscheibe (1). Damit ist die geleerte Dosierscheibe vom Luftstrom abgetrennt und stört nicht mehr.

### Beispiel 3

Anstelle der keilförmigen Nocken (45) und Schlitze (46) zum Transport der Dosierscheiben (1) kann auch ein an der Antriebsscheibe (44) angebrachter, exzentrisch angeordneter Stift (47) vorgesehen sein.
Der Stift (47) taucht in Dosierscheibenbohrungen (48) der jeweils nächsten Dosierscheiben (1) ein, wobei die Eindringtiefe durch eine Anordnung (51), (65), (66) gesteuert wird.
Hierbei ist der Kolben fest mit der Antriebsscheibe verbunden.

Bei einer Ausführungsvariante (nicht dargestellt) kann auf die Elemente (51), (65), (66) verzichtet werden. In der Anfangsstellung befindet sich die erste Dosierscheibe in einer toten Stellung und der Stift (47) greift in eine Dosierscheibenbohrung (48) der ersten Dosierscheibe (1) ein. Weiteres Eindringen des Stiftes (47) wird durch das Anstoßen des Stiftes (47) auf die zweite Dosierscheibe verhindert. Die vollen Dosierscheiben sind geringfügig gegeneinander verdreht, daß die Dosierscheibenbohrungen (48) nicht mehr genau fluchten.
Nachdem durch sukzessive Drehung der Kammern die erste Dosierscheibe entleert ist, fällt der Stift in die nun offenliegende Dosierscheiben-bohrung (48) der zweiten Dosierscheibe (1) ein und stellt eine Drehverbindung her. Der an der Oberseite des Kolbens (49) angebrachte Dichtring (52) sperrt die schon entleerten Dosierscheiben (1) vom Luftstrom ab.

### Beispiel 4 für einen Antriebsmechanismus

Anstelle des in seiner Eindringtiefe veränderlichen Kolbens (49) als Mittel zum Absperren der schon geleerten Dosierscheiben (1) kann auch eine Konstruktion verwendet werden, bei der die Luftdurchführungslöcher auf der äußeren Wand des Einwegdosierzylinders eine geringere Höhe als die Luftdurchführungslöcher der inneren Wand des Einwegdosierzylinders aufweisen.
Die Durchtrittsfläche der Dosierkammern nimmt daher von außen nach innen zu.
Bei der Anfangsanordnung liegen die Dosierscheiben (1) dicht aufeinander gepackt zusammen.
Die oberen Dosierscheibenvorsprünge (63) auf der Oberseite jeder Dosierscheibe (1) greifen in einen vertieften Bereich der Rille (64) auf der Unterseite der darüberliegenden Dosierscheibe ein, die Luftdurchführungsschlitze in der Außenwand des Zylinders sind nicht in Flucht mit den Dosierkammern angeordnet und damit sind die Dosierkammern (12) gesperrt.
Durch Drehung der Antriebsscheibe läuft der obere Dosierscheibenvorsprung (63) auf der Oberseite der Dosierscheibe aus einer Vertiefung in der Rille (64) auf der Unterseite der darüberliegenden Dosierscheibe (1) heraus und wird dadurch axial verschoben, daß die Luftdurchführungsöffnungen fluchten.

Nach dem Entleeren der Dosierkammern (12) einer Dosierscheibe (1) wird der obere Dosierscheibenvorsprung (63) in der Rille (64) blockiert und dreht so die zweite Dosierscheibe (1), die auf die gleiche Weise wie die erste Dosierscheibe (1) nach unten vor die Lufteintrittsöffnungen bewegt wird.

### Beispiel 5

In diesem Beispiel wird anhand eines nur beispielhaft dargestellten und keine erfindungsgemäße Querführung des Luftstroms für die Wirkstoffpartikel aufweisenden Inhalators eine Regelung des Luftstromes beschrieben, die auch in dem erfindungsgemäßen Inhalator zur Anwendung kommen kann. Diese Einrichtung besteht aus einem luftdruckgesteuerten Ventil (120), dessen Steuerorgan durch eine Membran (112) gebildet ist. Das Ventil (120) wird durch den Einatemluftstrom des Patienten resp. dem dadurch erzeugten Unterdruck gegenüber dem Atmosphärendruck gesteuert, derart, daß erst nach Erreichen einer bestimmten Druckdifferenz das Ventil (120) geöffnet wird. Diese Druckdifferenz stellt sicher, daß nach Öffnen des Ventil (120) eine für den Austrag des Pharmakons (113) ausreichende Strömungsgeschwindigkeit im Austragskanal (114) erreicht wird. Der durch das Öffnen des Ventils (120) bedingte plötzliche Druckausgleich resp. dem daraus resultierenden Strömungsimpuls wird die gleichmäßige Verteilung des Pharmakons (113) im einzuatmenden Luftstrom begünstigt.
In weiterer Funktion wirkt diese Einrichtung (120) als Rückschlagventil. Bei einer Fehlbenutzung durch einen Patienten kann es zu keinem Fehlaustrag des Pharmakons (113) kommen (z.B. wenn statt gesaugt geblasen wird).

Das Ventil (120) weist einen Ventilkörper (111) auf, welcher durch eine elastische Membran (112) mit einer definierten Kraft in Schließstellung (in Fig. 22 oben) gehalten wird. In dieser Stellung ist der Kanal (115) für den Mantelstrom (103) und der Austragskanal (114) für den Partikelaustragsstrom (101) verschlossen.

Durch Saugen eines Patienten am Mundstück des Inhalators wird im Kanal (115) ein Unterdruck aufgebaut, welcher auf die eine Membranseite (112 a) wirkt. Im Austragskanal (mundstückseitig) baut sich ebenfalls ein Unterdruck auf (Unterdruck in Fig. 22 mit - gekennzeichnet).
Die andere Seite (112 b) der Membran (112) steht über die ventilkörperseitige Gehäuseöffnung (116) mit der Atmosphäre in Verbindung.
Der Austragskanal (114) ist einlaßseitig durch eine Gehäuseöffnung (117) ebenso mit der Atmosphäre verbunden (Atmosphärendruck in Fig. 22 mit + gekennzeichnet).

Erreicht die Durckdifferenz die vorgesehene Größe, wird der Ventilkörper (111) durch die Membran (112) nach unten (in Fig. 23) bewegt, wobei das Ventil (120) geöffnet wird und ein Druckausgleich über den Austragskanal (114) und den Kanal (115) unter Austrag des Pharmakons (113) stattfindet.
Nach Austrag resp. nach Beendigung des Einatmens des Patienten (Druckausgleich) schließt die Membran (112) das Ventil (120).

Dieser Regelmechanismus kann in alle vorher beschriebenen beispielhaften Ausführungen des Inhalators eingebaut sein.

### Die Luftführung im Beispiel 1

Die Eintrittsöffnung (23) im Inhalatorboden (9) hat in der beispielhaften Ausführungsform der Erfindung einen Durchmesser von 7 mm. Der Luftstrom wird in drei Teilluftströme zerlegt:
1. durch den inneren Kanal (43) des inneren Dosierzylinders (4), (Falschluftstrom (102)),
2. durch die Längsnut (54) des äußeren Dosierzylinders (3), durch das Lufteinlaßloch (14) vor eine vorgefüllte Dosierkammer (12) der Dosierscheiben (1), und durch die vorgefüllte Dosierkammer (12), durch die Durchtrittsöffnung (16) in den inneren Kanal (43) des inneren Dosierzylinders (4), dort vereinigt sich der nun mit Wirkstoffpartikeln beladene Austragsstrom (101) mit dem Falschluftstrom (102), um durch den inneren Kanal (43) des inneren Dosierzylinders (4) nach oben zu strömen und durch die tangentialen Luftführungsschlitze (37) in die aus Zyklonkammergehäuse (6), Deckel (5) des inneren Dosierzylinders (4) und äußeren Dosierzylinders (3) gebildete Zyklonkammer (41) geleitet zu werden. An der Stelle des inneren Kanals (43) des inneren Dosierzylinders (4), an der durch die Durchtrittsöffnung (16) die mit Wirkstoffen oder mit einer Mischung aus Wirkstoffen und Hilfsstoffen beladene Luft in den inneren Kanal (43) eintritt, kann eine Verengung des inneren Kanals (43) angebracht sein, die in Form einer Venturi-Düse den Luftstrom beschleunigt und zu einem Druckabfall führt. Dieser Druckabfall im engsten Querschnitt der Venturi-Düse führt zu einer Beschleunigung des mit Wirkstoffen beladenen Luftstroms und trägt zu einer vollständigen Ausblasung des Wirkstoff aus der Dosierkammer (12) bei. In dieser Stelle der Luftführung werden die noch vorhandenen großen Aggregate aus Wirkstoffpartikeln oder Aggregate aus Hilfsstoff-und Wirkstoffpartikeln in lungengängige Partikel zerlegt.
3. durch die Längsnute (15), den Zwischenraum 56 und durch die Bohrungen (27) des Mundstücks (10) in die Austrittsöffnung (42) zwischen innerem und äußerem Mundstück zur Bildung des Mantelstroms (103). Der Mantelstrom umhüllt den partikeltragenden Luftstrom und hält ihn vom Mund-und Rachenraum fern.

Der Pulverinhalator kann aus einem medizinisch verträglichen Kunststoff angefertigt werden. Es kann zweckmäßig sein im Pulverinhalator die Unterbringung von Trockenmittel vorzusehen.

Der Gesamtdruckabfall bei der beispielhaften Ausführung des Inhalators beträgt bei einem Atemluftstrom von 60 Liter/Minute zwischen 50 und 150 mbar.
Beispielsweise beträgt der Druckabfall durch den ungefüllten Inhalator bei einem Atemluftstrom von 60 Liter/Minute 30 mbar, beim Verschließen des zentralen bypass 60 mbar und ohne den Mantelstrom 53 mbar.

## Patentansprüche

1. Pulverinhalator mit einer am dem Patienten zugewandten Ende eines Gehäuses angeordneten Mundstücköffnung (10 a) für mit Wirkstoff beladene Luft, mit einem Wirkstoffmagazin(1, 2) und mit Einrichtungen zum Einbringen des Wirkstoffes in einen durch den Inhalator geführten Luftstrom, wobei ein Gehäuseunterteil (8, 9) und ein Gehäuseoberteil (10, 11) gegeneinander beweglich ausgebildet sind, das Gehäuseoberteil (10, 11) ein Mundstück (10) mit der Mundstücköffnung (10a) aufweist, das Gehäuseunterteil hohlzylindrisch ausgebildet und eine Eintrittsöffnung (23) für Luft aufweist und im Gehäuseunterteil (8, 9) konzentrisch ein Zylinder vorhanden ist,
dadurch gekennzeichnet, daß das Gehäuseunterteil (8, 9) in das Gehäuseoberteil (10, 11) in Achsrichtung einschiebbar ist und um seine Längsachse im Gehäuseoberteil (10, 11) drehbar ist, der im Gehäuseunterteil (8, 9) angeordnete Zylinder einen äußeren Dosierzylinder (3), formschlüssig verbunden mit einem inneren Dosierzylinder (4) mit geringerem Durchmesser aufweist, so daß ein Zwischenraum (53) ausgebildet ist, in dem das hohle, Dosierkammern (12) enthaltende Wirkstoffmagazin (1, 2) Platz findet, der innere Dosierzylinder (4) eine Durchtrittsöffnung (16) zum Zwischenraum (53) aufweist, die Eintrittsöffnung (23) in dem Gehäuseunterteil in direkter durchgehender Verbindung mit einem inneren Kanal (43) des inneren Dosierzylinders (4) steht, am oberen Ende des inneren Kanals (43) tangentiale Luftführungsschlitze (37) für mit Wirkstoff beladene Luft zu einer Zyklonkammer (41) vorhanden sind, die Zyklonkammer (41) mit einer Öffnung (17) zu einem Ausströmrohr (29) des Mundstücks (10) verbunden ist, Einrichtungen vorhanden sind, die ein axiales Verschieben und/oder Drehen des Wirkstoffmagazins (1, 2) um seine Längsachse im Zwischenraum (53) ermöglichen, ein mit dem Mundstück (10) verbundener Führungszylinder (7) mit größerem Außendurchmesser den äußeren Dosierzylinder (3) umgibt, in der Außenwand des äußeren Dosierzylinders (3) eine der Luftführung dienende Längsnut (54) vom unteren Ende bis zu einem etwa in der Mitte des äußeren Dosierzylinders (3) angeordneten Lufteinlaßloch (14) in den Zwischenraum (53) vorhanden ist, das Lufteinlaßloch (14) zu der Durchtrittsöffnung (16) des inneren Dosierzylinders (4) fluchtend angeordnet ist und ein axiales Verschieben und/oder Verdrehen des Wirkstoffmagazins (1, 2) jeweils eine der darin angeordneten Dosierkammern (12) vor Lufteinlaßloch (14) und Durchtrittsöffnung (16) befördert und daß ferner von der Eintrittsöffnung (23) über eine der Luftführung dienende Längsnut (15) auf der Außenseite des äußeren Dosierzylinders (3) eine durchgehende Verbindung bis zu einer einen Mantelluftstrom erzeugenden Austrittsöffnung (42) im Mundstück (10) vorhanden ist.

2. Pulverinhalator gemäß Anspruch 1, dadurch gekennzeichnet, daß die Einrichtungen, die ein axiales Verschieben und/oder Drehen des Wirkstoffmagazins (1, 2) um seine Längsachse im Zwischenraum (53) ermöglichen, aus Führungsnuten (18) und Steuerkurven (19) auf der Außenseite des Führungszylinders (7), einer Führungsnocke (21) an einem Außenzylinder (8) des Gehäuseunterteils sowie Furchnuten (8 a), die auf der Innenseite des Außenzylinders (8) angebracht sind, bestehen.

3. Pulverinhalator gemäß Anspruch 2, dadurch gekennzeichnet, daß die Steuerkurven (19) so ausgebildet sind, daß nach dem Leeren aller Dosierkammern (12) in einem aus Dosierscheiben (1) und Endscheibe (2), innerem Dosierzylinder (4) und äußerem Dosierzylinder (3) sowie Zyklonkammergehäuse (6) gebildeten Einwegdosierzylinder (55) das Gehäuseunterteil (8, 9) nach Entfernen des Gehäuseoberteils (10, 11) des Pulverinhalators vom Führungszylinder (7) in axialer Richtung trennbar ist.

4. Pulverinhalator gemäß Anspruch 3, dadurch gekennzeichnet, daß die Dosierscheiben (1) des Wirkstoffmagazins (1, 2) mit Mitteln versehen sind, die einen gegenseitigen Antrieb der Dosierscheiben (1) erlauben.

5. Pulverinhalator gemäß Anspruch 4, dadurch gekennzeichnet, daß die Dosierscheiben (1) sich mittels Nocken (45) und Schlitzen (46) antreiben und vor Luftdurchführungsöffnungen (62) geführt werden.

6. Pulverinhalator gemäß Anspruch 4, dadurch gekennzeichnet, daß die einzelnen Dosierscheiben (1) des Wirkstoffmagazins (1, 2) durch einen in Dosierscheibenbohrungen (48) in den jeweils nächsten Dosierscheiben eindringenden Stift (47) bewegt werden.

7. Pulverinhalator gemäß Anspruch 6, dadurch gekennzeichnet, daß die Dosierkammern (12) zwischen den Dosierscheiben (1) einen sich in Richtung des inneren Dosierzylinders (4) vergrößernden Querschnitt haben.

8. Pulverinhalator gemäß den Ansprüchen 1 bis 7, dadurch gekennzeichnet, daß zwischen der Zyklonkammer (41) und der Mundstücköffnung (10 a) am Gehäuseoberteil eine Mundstückhülle (11) angeordnet ist, worin sich das Ausströmrohr (29) für mit Wirkstoff beladene Luft befindet und um dieses Ausströmrohr (29) konzentrisch ein Ringkanal (58) für die Mantelstromluft angeordnet ist.

9. Pulverinhalator gemäß dem Anspruch 8, dadurch gekennzeichnet, daß das Ausströmrohr (29) einen Mundstückkanal (57) mit in Luftstromrichtung sich vergrößerndem Durchmesser aufweist.

10. Pulverinhalator gemäß den vorstehenden Ansprüchen, dadurch gekennzeichnet, daß die Mengen der zu verabreichenden Stoffmischung aus biologisch aktiven Substanzen und Hilfsstoffen in einer Dosierkammer (12) zwischen 0,05 mg und 50 mg liegen.

11. Pulverinhalator gemäß den vorstehenden Ansprüchen, dadurch gekennzeichnet, daß die Mengen der zu verabreichenden biologisch aktiven Substanzen in einer Wirkstoffkammer (12) zwischen 0,025 mg und 50 mg liegen.

12. Pulverinhalator nach einem der vorstehenden Ansprüche 8 - 11, dadurch gekennzeichnet, daß er anstelle der geraden Mundstückhülle (11) ein gebogenes Mundstück (60) aufweist.

13. Pulverinhalator gemäß einem oder mehrerer vorstehender Ansprüche, dadurch gekennzeichnet, daß der innere Kanal (43) gegenüber der Durchtrittsöffnung (16) eine Verengung des Querschnitts aufweist.

14. Pulverinhalator gemäß einem oder mehrerer vorstehender Ansprüche, dadurch gekennzeichnet, daß er eine Einrichtung zur Regelung des Luftstroms aufweist.

15. Pulverinhalator gemäß Anspruch 14, dadurch gekennzeichnet, daß die Regelung des Luftstroms durch eine Einrichtung, bestehend aus Ventilkörper (111) und elastischer Membran (112) erfolgt.

## Claims

1. Powder inhaler having a nozzle opening (10 a) for air charged with active substance, arranged at the end of a case and directed towards the patient, and having a storage chamber (1, 2) for the active substance and having devices for introducing the active substance into an air stream passed through the inhaler, with a lower part of the case (8, 9) and an upper part of the case (10, 11) being constructed movable against one another, the upper part of the case (10, 11) possesses a nozzle (10) having the nozzle opening (10 a), the lower part of the case is constructed as a hollow cylinder and possesses an inlet (23) for air and there is a cylinder placed concentrically in the lower part of the case (8, 9), characterised in that the lower part of the case (8, 9) is extendable in the axial direction into the upper part of the case (10, 11) and is rotatable around its longitudinal axis in the upper part of the case (10, 11), the cylinder arranged in the lower part of the case (8, 9) possesses an outer dosing cylinder (3), interlocking with an inner dosing cylinder (4) having a smaller diameter, so that a space (53) is formed, wherein there is room for the storage chamber (1, 2) for the active substance containing the hollow, dosing chambers (12), the inner dosing cylinder (4) has a passage opening (16) to the space (53), the inlet (23) in the lower part of the case is in direct continuous connection with an inner duct (43) of the inner dosing cylinder (4), at the upper end of the inner duct (43) there are tangential air conduction slits (37) for the air laden with active substance to a cyclone chamber (41), the cyclone chamber (41) is connected via an opening (17) to an ejector tube (29) of the nozzle (10), there are devices which render possible in the space (53) an axial displacement and/or rotation of the storage chamber (1, 2) for the active substance around its longitudinal axis, a guide cylinder (7) connected with the nozzle (10) and having a larger outer diameter surrounds the outer dosing cylinder (3), in the outer wall of the outer dosing cylinder (3) there is a longitudinal slot (54) serving to conduct air from the lower end up to an air inlet hole (14) arranged approximately in the middle of the outer dosing cylinder (3) into the space (53), the air inlet hole (14) is arranged in alignment with the passage opening (16) of the inner dosing cylinder (4) and an axial displacement and/or rotation of the storage chamber (1, 2) for the active substance conveys in each case one of the dosing chambers (12) arranged therein in front of air inlet hole (14) and passage opening (16) and that in addition, from the inlet (23) via a longitudinal slot (15) serving to conduct air onto the outer side of the outer dosing cylinder (3), there is a continuous connection up to an outlet (42), which produces a sheathing air stream in the nozzle (10).

2. Powder inhaler according to claim 1, characterised in that the devices which render possible in the space (53) an axial displacement and/or rotation of the storage chamber (1, 2) for the active substance around its longitudinal axis consist of guide slots (18) and radial cams (19) on the outer side of the guide cylinder (7), of a cam follower (21) along an outer cylinder (8) of the lower part of the case as well as grooves (8a), which are positioned on the inner side of the outer cylinder (8).

3. Powder inhaler according to claim 2, characterised in that the radial cams (19) are constructed in such a way that after the emptying of all the dosing chambers (12) in a disposable dosing cylinder (55) comprising dosing discs (1) and end disc (2), inner dosing cylinder (4), outer dosing cylinder (3) and cyclone chamber case (6), the lower part of the case (8, 9) is separable in the axial direction, after the removal from the guide cylinder (7) of the upper part of the case (10, 11) of the powder inhaler.

4. Powder inhaler according to claim 3, characterised in that the dosing discs (1) of the storage chamber (1, 2) for the active substance are provided with means of permitting an interaction of the dosing discs (1).

5. Powder inhaler according to claim 4, characterised in that the dosing discs (1) are operated by means of cams (45) and slots (46) and are passed in front of openings (62) for the passage of air.

6. Powder inhaler according to claim 4, characterised in that the individual dosing discs (1) of the storage chamber (1, 2) for the active substance are moved by a pin (47) within holes (48) in the dosing discs, which pin penetrates into each of the following dosing discs.

7. Powder inhaler according to claim 6, characterised in that the dosing chambers (12) between the dosing discs (1) have a transverse section increasing in the direction of the inner dosing cylinder (4).

8. Powder inhaler according to claims 1 to 7, characterised in that between the cyclone chamber (41) and the nozzle opening (10 a) in the upper part of the case there is arranged a nozzle cover (11), wherein there is positioned the ejector tube (29) for air charged with active substance, and around this ejector tube (29) an annular duct (58) for the sheathing air stream is arranged concentrically.

9. Powder inhaler according to claim 8, characterised in that the ejector tube (29) possesses a nozzle duct (57) having a diameter increasing in the direction of the air stream.

10. Powder inhaler according to the preceding claims, characterised in that the quantities of the mixture of substances to be administered, comprising biologically active substances and auxiliary substances, contained in a dosing chamber (12) are between 0.05 mg and 50 mg.

11. Powder inhaler according to the preceding claims, characterised in that the quantities of the biologically active substances to be administered contained in a chamber (12) for the active substances are between 0.025 mg and 50 mg.

12. Powder inhaler according to one of the preceding claims 8 to 11, characterised in that it possesses a curved nozzle (60) instead of the straight nozzle cover (11).

13. Powder inhaler according to one or more of the preceding claims, characterised in that the inner duct (43) has a restriction in the transverse section opposite the passage opening (16).

14. Powder inhaler according to one or more of the preceding claims, characterised in that it possesses a device for controlling the air stream.

15. Powder inhaler according to claim 14, characterised in that the control of the air stream is effected by a device consisting of a valve body (111) and elastic membrane (112).

## Revendications

1. Inhalateur de poudres comportant un orifice d'embout (10a) prévu à l'extrémité d'un boîtier du côté du patient, pour de l'air chargé de matière active, un magasin de matière active (1, 2) et des installations pour introduire la matière active dans un courant d'air traversant l'inhalateur, une partie inférieure (8, 9) du boîtier et une partie supérieure (10, 11) du boîtier étant réalisées mobiles l'une par rapport à l'autre, la partie supérieure (10, 11) du boîtier comportant l'embout (10) avec l'orifice d'embout (10a), la partie inférieure du boîtier étant en forme de cylindre creux avec un orifice d'entrée (23) pour l'air et un cylindre concentrique prévu dans la partie inférieure (8, 9) du boîtier, caractérisé en ce que la partie inférieure (8, 9) du boîtier peut être glissée axialement dans la partie supérieure (10, 11) du boîtier et être mobile en rotation autour de son axe longitudinal dans la partie supérieure (10, 11) du boîtier, le cylindre prévu dans la partie inférieure (8, 9) du boîtier se composant d'un cylindre de dosage extérieur (3) relié par une liaison par la forme avec un cylindre de dosage intérieur (4) de plus petit diamètre, pour former un volume intermédiaire (53) logeant le magasin de matière active (1, 2) creux, comportant les chambres de dosage (12), et le cylindre de dosage intérieur (4) comporte un orifice de passage (16) vers le volume intermédiaire (53), l'orifice d'entrée (23) dans la partie inférieure du boîtier étant en communication continue, directe avec un canal intérieur (43) du cylindre de dosage intérieur (4), et à l'extrémité supérieure du canal intérieur (43), il y a des fentes de guidage d'air (37), tangentielles pour de l'air chargé de matière active vers une chambre de cyclone (41), cette chambre de cyclone (41) étant munie d'un orifice (17) vers un tube de sortie (29) de l'embout (10), des installations étant prévues pour permettre un coulissement axial et/ou une rotation du magasin de matière active (1, 2) autour de son axe longitudinal dans le volume intermédiaire (53), un cylindre de guidage (7) étant relié à l'embout (10) de plus grand diamètre extérieur pour entourer le cylindre de dosage extérieur (3), et dans la paroi extérieure du cylindre de dosage extérieur (3), il est prévu une rainure longitudinale (54) servant au guidage de l'air et allant de l'extrémité inférieure jusqu'à un orifice d'entrée d'air (14) prévu sensiblement au milieu du cylindre de dosage extérieur (3), dans le volume intérieur (53), l'orifice d'entrée d'air (14) étant aligné sur l'orifice de passage (16) du cylindre de dosage intérieur (4) et un coulissement axial et/ou une rotation du magasin de matière active (1, 2) mettant l'une des chambres de dosage (12) qui s'y trouve devant l'orifice d'entrée d'air (14) et l'orifice de passage (16) et en ce qu'en outre une rainure longitudinale (15) servant au guidage de l'air relie l'orifice d'entrée (23) au côté extérieur du cylindre de dosage extérieur (3), en établissant une liaison continue jusqu'à un orifice de sortie (42) prévu dans l'embout (10), et créant un courant d'air-enveloppe.

2. Inhalateur de poudres selon la revendication 1, caractérisé en ce que les installations, qui permettent un coulissement axial et/ou une rotation du magasin de matière active (1, 2) autour de son axe longitudinal dans le volume intermédiaire (53) se composent de rainures de guidage (18) et de courbes de commande (19) sur le côté extérieur du cylindre de guidage (7), d'une came de guidage (21) sur un cylindre extérieur (8) de la partie inférieure du boîtier ainsi que des rainures en forme de sillons (8a) prévues sur le côté intérieur du cylindre extérieur (8).

3. Inhalateur de poudres selon la revendication 2, caractérisé en ce que les courbes de commande (19) sont réalisées pour qu'après vidage de toutes les chambres de dosage (12) dans un cylindre de dosage jetable (55) d'un cylindre de dosage intérieur (4) formé de disques de dosage (1) et d'un disque d'extrémité (2) du cylindre de dosage intérieur (4) et du cylindre de dosage extérieur (3) ainsi que du boîtier (6) formant la chambre de cyclone, la partie inférieure (8, 9) du boîtier peut se séparer dans la direction axiale après séparation de la partie supérieure (10, 11) du boîtier de l'inhalateur de poudres du cylindre de guidage (7).

4. Inhalateur de poudres selon la revendication 3, caractérisé en ce que les disques de dosage (1) du magasin de matière active (1, 2) sont munis de moyens, permettant un entraînement réciproque des disques de dosage (1).

5. Inhalateur de poudres selon la revendication 4, caractérisé en ce que les disques de dosage (1) s'entraînent à l'aide de cames (45) et de fentes (46) et sont conduites devant les orifices de passage d'air (62).

6. Inhalateur de poudres selon la revendication 4, caractérisé en ce que les différents disques de dosage (1) du magasin de matière active (1, 2) sont déplacés par une tige (47) pénétrant dans les perçages de disques de dosage (48) correspondant aux disques de dosage respectivement suivants.

7. Inhalateur de poudres selon la revendication 6, caractérisé en ce que les chambres de dosage (12) présentent entre les disques de dosage (1), une section augmentant dans la direction du cylindre de dosage intérieur (4).

8. Inhalateur de poudres selon les revendications 1 à 7, caractérisé en ce qu'entre les chambres à cyclone (41) et l'orifice d'embout (10a) sur la partie supérieure du boîtier, il est prévu une enveloppe d'embout (11) munie du tube de sortie (29) pour l'air chargé de ma-tière active, et concentriquement autour de ce tube de sortie (29), il y a un canal annulaire (58) pour le courant d'air-enveloppe.

9. Inhalateur de poudres selon la revendication 8, caractérisé en ce que le tube de sortie (29) comporte un canal d'embout (57) d'un diamètre augmentant dans la direction de l'écoulement d'air.

10. Inhalateur de poudres selon les revendications précédentes, caractérisé en ce que la quantité de mélange de matières à fournir se compose de substances actives biologiquement et de matières auxiliaires placées dans une chambre de dosage (12), les quantités étant comprises entre 0,05 mg et 50 mg.

11. Inhalateur de poudres selon les revendications précédentes, caractérisé en ce que les quantités de substances biologiquement actives à traiter dans les chambres actives (12) sont comprises entre 0,025 mg et 50 mg.

12. Inhalateur de poudres selon l'une des revendications précédentes 8 à 11, caractérisé en ce qu'à la place de l'enveloppe d'embout (11) droite, il est prévu un embout cintré (60).

13. Inhalateur de poudres selon l'une ou plusieurs des revendications précédentes, caractérisé en ce que le canal intérieur (43) présente un rétrécissement de section par rapport à l'orifice de passage (16).

14. Inhalateur de poudres selon l'une ou plusieurs des revendications précédentes, caractérisé par une installation pour réguler le courant d'air.

15. Inhalateur de poudres selon la revendication 14, caractérisé en ce que la régulation du courant d'air se fait par une installation composée d'un corps de soupape (111) et d'une membrane élastique (112).
